# EUROPEAN PATENT APPLICATION

(11) **EP 1 880 740 A2**
(43) Date of publication of application: **23.01.2008**
(21) Application number: 07013522.3
(22) Date of filing: 10.07.2007
(51) Int. Cl.: A61M 1/36

(54) **Connector and blood line**

(30) Priority: 18.07.2006 JP 2006195503; 27.03.2007 JP 2007083100
(71) Applicant: TERUMO KABUSHIKI KAISHA, Tokyo 151-0072 (JP)
(72) Inventor: Mitsuaki, Ogihara, Fujinomiya-shi Shizuoka 418-0015 (JP); Masaaki, Abe, Fujinomiya-shi Shizuoka 418-0015 (JP); Eiji, Kato, Fujinomiya-shi Shizuoka 418-0015 (JP)
(74) Representative: Casalonga, Axel

(57) **Abstract**

A blood line assembly which is capable of being handled relatively easily includes a first tube, a second tube having a second tube body and two branch tubes branching from the second tube body, and a connector having plugs on which the first and branch tubes are removably fit. In the blood line assembly, by removing the first tube and the branch tubes from the connector, the first tube and the branch tubes can be connected to respective catheters positioned in a patient.

## Description

The disclosed subject matter generally pertains to blood lines having useful application in an extracorporeal circuit. More specifically, the subject matter pertains to a connector and a blood line.

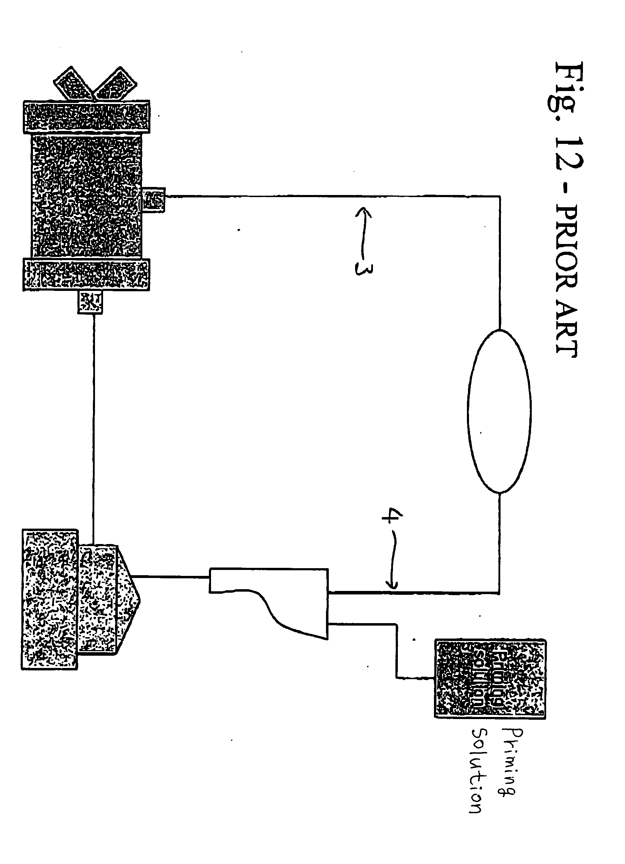

As illustrated in Fig. 12, a traditional blood line for extracorporeal circulation uses an integrated line that includes venous and arterial continuous at the connections to a patient. This configuration is used to facilitate performance of a priming operation. After the priming, the line is cut at the relevant point, for example by a cutter, and then connected to the patient. In this manner, the traditional blood line is somewhat troublesomeness in that it involves a cutting operation through use of a cutter. Also, because the integrated continuing portion is in the form of a loop, the blood line before use is problematically bulky when it is received within a package.

According to one aspect, a blood line assembly comprises a first tube possessing an end, a second tube comprised of a second tube body possessing an end and two branch tubes branching from the end of the second tube body, with each of the two branch tubes possessing an end, and a connector comprising first, second and third connections each provided with a through passage, with the end of the first tube being removably fitted to the first connection, and the end of each of the branch tubes being removably fitted to the second and third connections respectively. The first tube and the branch tubes are removable from the respective connections of the connector for subsequent connection to respective catheters positioned in a patient.

According to another aspect, a connector comprises a connector body possessing a lumen, a plurality of connections provided on the connector body, with each connector possessing a through passage communicating with the lumen of the connector body and with each of the connections being adapted to be removably connected to a respective tube to communicate a lumen of each tube to the lumen of the connector body, and opening-and-closing means for opening and closing the passages of all of said plurality of connections together. The opening-and-closing means is operable to selectively position the connector in a communication state in which the passages of all of said plurality of connections are in communication with the lumen and a non-communication state in which the passages of all of said plurality of connections are blocked from communicating with the lumen.

In accordance with a further aspect, a method of using a blood line comprises connecting a blood line assembly to a source of priming liquid.
The blood line assembly comprises a connector, a first tube and a second tube, with one of the first and second tubes being comprised of a tube body and two branch tubes branching from the tube body, and the connector comprising first, second and third connections each provided with a through passage, with the first tube being removably fitted to the first connection and each of the branch tubes being removably fitted to the second and third connections respectively. The method also comprises introducing the priming liquid from the priming liquid source into the first tube so that the priming liquid in the first tube flows into the second tube by way of the connector, disconnecting the first tube from the first connection and disconnecting each of the branch tubes from the second and third connections respectively, and connecting the first tube and the branch tubes to a respective catheter positioned in a patient.

With the blood line assembly, connector and method disclosed here, it is possible to provide a blood line that is relatively easy to prepare for extracorporeal circulation of blood without the need for cutting tubing.

Fig. 1 is a schematic diagram showing an extracorporeal circuit including a blood line as disclosed herein.

Fig. 2 is an enlarged perspective view of the first and second tubes forming a part of the blood line illustrated in Fig. 1 together with the connector.

Fig. 3 is a perspective view showing the first and second tubes of the blood line of Fig. 1, and the connector, received in a package.

Figs. 4A and 4B are cross-sectional views taken along the section line IV-IV in Fig. 2, with Fig. 4A depicting a communication state of the connector and Fig. 4B depicting a non-communication state of the connector.

Figs. 5A and 5B are cross-sectional views taken along the section line V-V in Fig. 2, with Fig. 5A illustrating a communication state of the connector and Fig. 5B illustrating a non-communication state of the connector.

Fig. 6 is a cross-sectional view taken along the section line VI-VI in Fig. 2.

Figs. 7A and 7C are perspective views showing a second embodiment of a connector disclosed herein, with Fig. 7A illustrating a communication state and Fig. 7B illustrating a non-communication state.

Fig. 8 is a perspective view showing another example of a through-hole.

Fig. 9 is a longitudinal cross-sectional view of a third embodiment of a connector disclosed herein.

Fig. 10 is a perspective view of a fourth embodiment of a connector.

Fig. 11 is a perspective view of a fifth embodiment of a connector disclosed herein.

Fig. 12 is a schematic illustration of a prior art blood line.

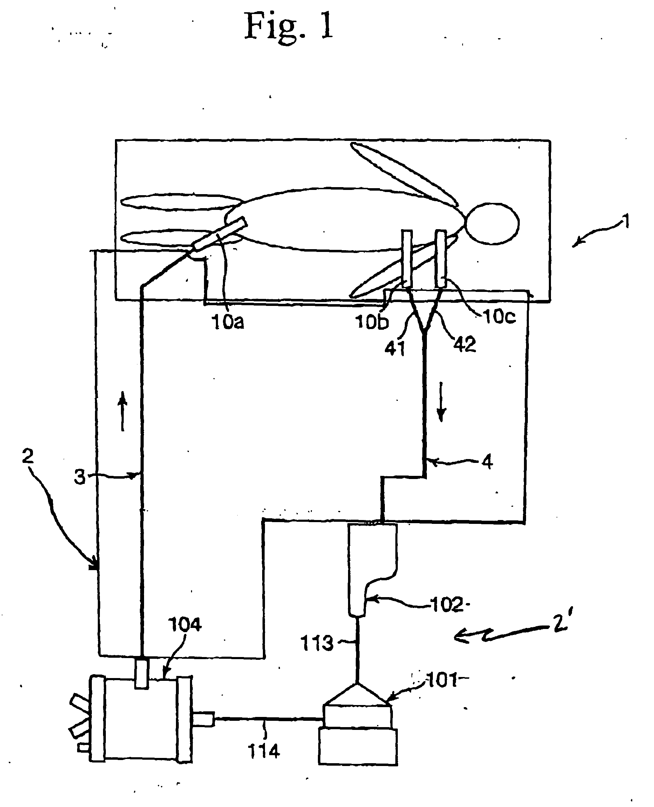

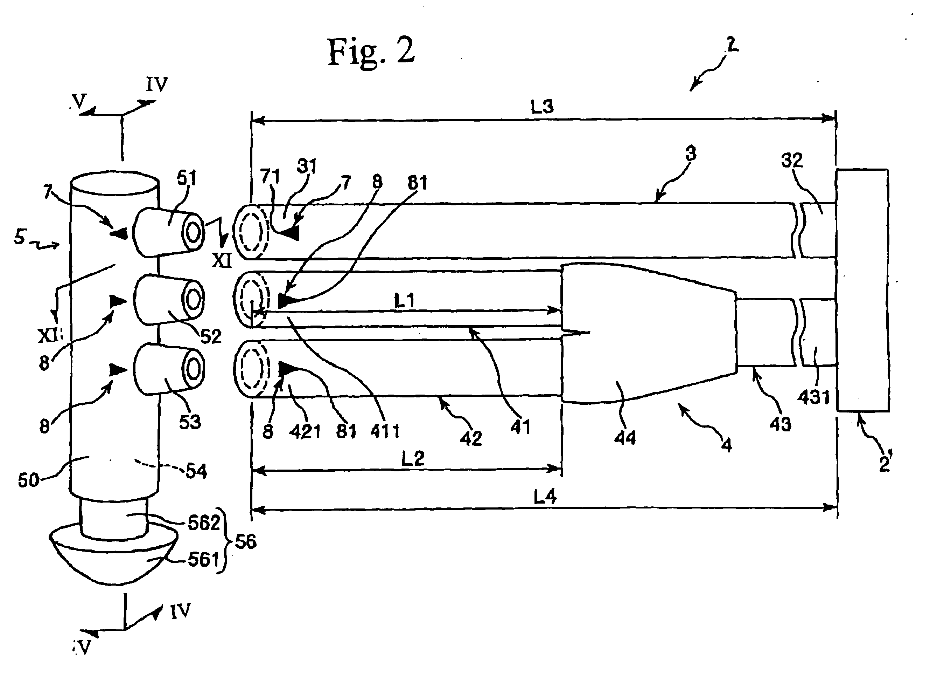

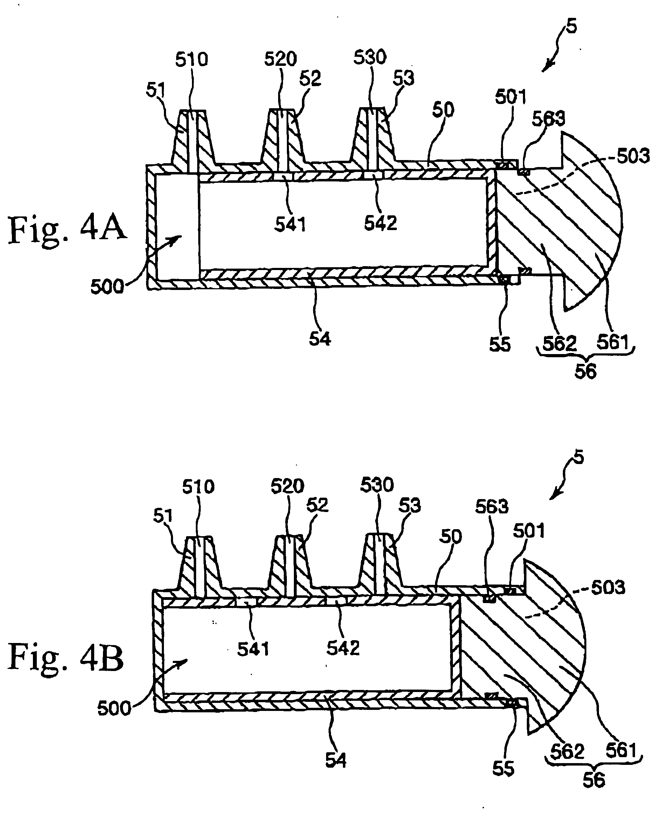

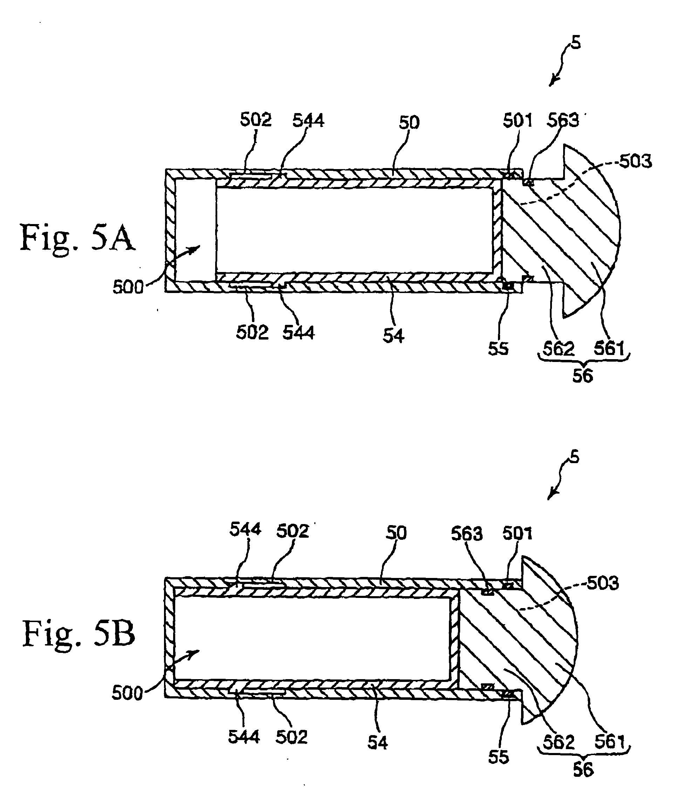

Fig. 1 schematically illustrates an extracorporeal circuit 1 used to circulate the blood of a patient through connection to several catheters 10a, 10b, 10c retained in the patient. In the description below referring to Figs. 2, 4 and 5, the left side is referred to as a "tip" while the right side is referred to as a "base". In Figs. 4 and 5, the upper side is referred to as the "upper" side while the lower side is taken as the "lower" side.

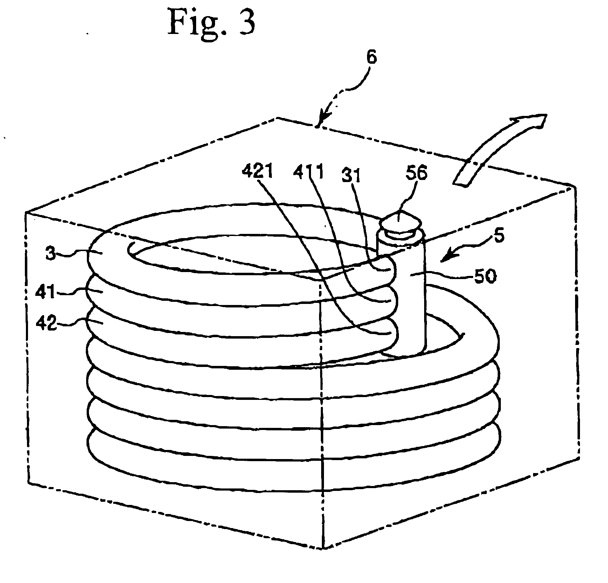

As shown in Figs. 1 and 2, a blood line 2 includes first and second tubes 3, 4. The first tube 3 is connected to a catheter 10a positioned in a patient and the second tube 4 is connected to a pair of catheters 10b, 10c also positioned in a patient. The second tube 4 is connected to the catheters 10b, 10c by way of branch tubes 41, 42. As described in more detail below, the first and second tubes 3, 4 are attached or connected (at the tip 31 and at the branch tube 41, 42 tips 411, 421) to a connector 5 before being connected to the respective catheters 10a, 10b, 10c (see Figs. 2 and 3).

In the extracorporeal circuit 1, a blood reservoir 102, a pump 101 and an oxygenator 104 are arranged in that order from the upstream end, thus being connected in a line through several tubes 113, 114. This arrangement of medical devices 102, 113, 101, 114, 104 are collectively identified as 2' in Fig. 1.

In the extracorporeal circuit 1 thus arranged, the blood from the patient enters the blood reservoir 102 from the second tube 4, from which the blood is sent by the pump 101 to the oxygenator 104 where oxygen is added, and then the oxygenated blood is returned to the patient.

The first tube 3 is formed as an elongated tubular element (tube) possessing flexibility. The first tube 3 has a base 32 connected to a blood outlet port of the oxygenator 104.

During extracorporeal blood circulation, the tip 31 (see Fig. 2) of the first tube 3 is connected to the catheter 10a as shown in Fig. 1. On the other hand, when the first tube 3 is packaged in a package 6 shown in Fig. 3 which is described in more detail below, and during priming, the tip end 31 of the first tube 3 is connected to the connector 5 shown in Fig. 2. During use, the first tube 3 is referred to as an arterial line.

As illustrated in Fig. 2, the tip 31 of the first tube 3 is provided with a first label (marking) 7 specifying that the tube is the first tube or is for use as an arterial line as shown in Fig. 2. In the illustrated embodiment, the first label 7 is triangular in form (an isosceles triangular form) indicating the blood-flow direction. That is, the triangular-shaped first label includes an apex 71 closer to the tip 31 and pointing in the direction of the tip 31, i.e., closer to the patient when the tip 31 of the first tube 3 is connected to the catheter 10a, thus indicating a direction of flow towards the tip 31.

By virtue of the first label 7, the first tube 3 is able to be connected, rapidly and positively to the catheter 10a previously positioned in the patient during set-up of an extracorporeal circuit by connecting the blood line 2 with the patient. Thus, the first tube 3 (blood line 1) is relatively easy to handle and appropriately connect.

The material forming the first tube is not particularly limited. As an example, the material can be a flexible polymeric material, e.g., a non-rigid polyvinyl chloride.

The second tube 4 comprises a second tube body 43 and two branch tubes 41, 42 that branch from the second tube body 43 through a bifurcation 44 provided at the tip of the second tube body 43. The second tube body 43 is formed as an elongated tubular element (tube) which possesses flexibility. The base end of the second tube body 43 is connected to the blood reservoir 102 as shown in Fig. 1. The second tube 4 is referred to as a venous line.

The bifurcation 44 at the tip of the second tube body 43 is preferably V-shaped or Y-shaped. Through the bifurcation 44, the second tube body 43 communicates with each of the branch tubes 41, 42. The branch tubes 41, 42 are each formed as an elongated tubular element (tube) possessing flexibility.

During extracorporeal blood circulation, the tip 411 (see Fig. 2) of the branch tube 41 is connected to the catheter 10b as shown in Fig. 1. On the other hand, in the packaged state shown in Fig. 3 when the branch tube 41 is packaged in the package 6, and during priming, the tip 411 of the branch tube 41 is connected to the connector 5.

During extracorporeal blood circulation, the tip 421 (see Fig. 2) of the branch tube 42 is connected to the catheter 10c as shown in Fig. 1. On the other hand, in the packaged state shows in Fig. 3 when the branch tube 42 is packaged in the package 6, and during priming, the tip 421 of the branch tube 42 is connected to the connector 5.

As shown in Fig. 2, the branch tube 41 has a length L1 equal to a length L2 of the branch tube 42, it being understood that the term equal is inclusive of near equality. Thus, when the branch tubes 41, 42 are laid out in flat and taught condition, the tips 411, 412 are positioned lengthwise of the second tube 4 at the same position.

The tip 411, 421 of each branch tube 41, 42 is provided with a second label (marking) 8 specifying that the tube is the second tube or is for use as a venous line as shown in Fig. 2. In the illustrated embodiment, the second label 8 is triangular in form (an isosceles triangular form) indicating the blood-flow direction. That is, the triangular-shaped second label includes an apex 81 pointing towards the base.

By virtue of the second label 8, when the blood line 2 is connected to the patient during set-up of an extracorporeal circuit, the branch tubes 41, 42 are able to be relatively rapidly and positively connected to the catheters 10b, 10c respectively previously positioned in the patient. Thus, the second tube 4 is relatively easy to handle and appropriately connect.

As shown in Fig. 2, the first tube 3 has an overall length L3 equal to the overall length L4 of the second tube 4, it being understood that the term equal is inclusive of near equality. Thus, when the first and second tubes 3, 4 are laid out in a flat and taught condition, the tips 31, 411, 421 are at the same position lengthwise of the first tube 3.

By making the lengths L3, L4 equal, the first and second tubes 3, 4 are less likely to slacken when bundled together. If the lengths L3, L4 differ from one another and the first and second tubes 3, 4 are bundled together, a slackening can result which degrades the appearance. In addition, should a tube kink occur, it is possible that a local decrease of blood-passage cross-sectional area could arise, making it quite difficult to carry out sterilization due to non-transmission of the sterilizing gas.

The material forming the second tube body 43 (second tube element), inclusive of the bifurcation 44, and the branch tubes 42, 43 is not limited. By way of example, the same material as that used to fabricate the first tube can be employed.

When the blood line 2 is packaged in the package 6 together with the connector 5, and during priming, the connector 5 is preferably attached to the first tube 3 and to the branch tubes 41, 42 of the second tube 4. After priming, and before connecting the first and second tubes 3, 4 to the respective catheters 10a, 10b, 10c, the connector 5 is removed from the first and second tubes 3, 4.

The blood line 2 is received in (packaged) in the package 6 together with the connector 5 as shown in Fig. 3. The first and second tubes 3, 4, attached to the connector 5, are bundled together in a helical form, with the tubes 3, 4 being wound in the same winding direction in the package 6.

By virtue of this packaging arrangement, the first and second tubes 3, 4 can be easily taken out of the package 6 by holding the connector 5 and pulling it in the direction of the arrow shown in Fig. 3. After removing the first and second tubes 3, 4 from the packaging, the first and second tubes 3, 4 are released from the wound condition into a natural state, i.e., into a state free of kinks or twists. This prevents the tubes from being closed due to the occurrence of kink or twist.

As shown in Figs. 2 and 4-6, the connector 5 comprises a first cylinder or hollow member 50, three outwardly extending plugs or connections 51, 52, 53, and a second cylinder or movable member 54. The connections 51, 52, 53 extend outwardly from the first cylinder 50 in a direction nearly vertical to the longitudinal axis of the first cylinder 50. The second cylinder 54, forming a received member, is positioned in the first cylinder 50.

As shown in Figs. 4A, 4B, 5A and 5B, the first cylinder 50 is constructed as a connector body formed by a bottomed cylindrical member closed at one end (base end) and open at the opposite end. The first cylinder 50 has a lumen 500 therein.

In the illustrated embodiment, the first cylinder 50 has a shell or wall that is cylindrical in form. The shell or wall of the first cylinder 50 need not be cylindrical in form, but may alternatively configured such a polygonal cylindrical form, e.g., quadrilateral, hexagonal in cross-section.

As mentioned, the end of the first cylinder 50 opposite the closed end is open in that it is provided with an opening 503. The inner peripheral surface of the first cylinder 50 in an area adjacent the opening 503 is provided with a groove 501 that is ring-shaped or annular in form. An O-ring (ring-formed resilient member) 55 is positioned in the groove 501. The O-ring 55 is adapted to closely contact the outer peripheral surface of the second cylinder 54 and the outer peripheral surface of a connection 562 of a manipulator 56, described in more detail below. This helps ensure liquid-tightness at the inside of the connector 5 (lumen 500). The O-ring 55 maintains the liquid-tightness in the connector 5 regardless of the displacement or amount of movement of the second cylinder 54 and the connection 562 of the manipulator 56.

The material forming the O-ring 55 is not necessarily limited, though is preferably a resilient material, for example natural rubber or isoprene rubber.

The first cylinder 50 is preferably made substantially transparent (transparent and colorless, transparent and colored, or semi-transparent).
This permits visual observation of the tip of the second cylinder 54 and the position of the through-holes 541, 542, described in more detail below, through the first cylinder 50. This helps facilitate the change-over of the connector 5 between a communication state, as shown in Fig. 4A, and a non-communication state, shown in Fig. 4B.

As mentioned above, the shell of the first cylinder 50 is provided with three plugs or connectors 51, 52, 53 that are preferably formed integrally and in one-piece with the first cylinder 50. The plugs 51, 52, 53 are arranged intermittently on the first cylinder 50, meaning that they are spaced apart from one another at equal intervals along the lengthwise or axial extent of the first cylinder 50.

As shown in Fig. 2, the tip 31 of the first tube 3 is removably fitted to the plug 51, the tip 411 of the branch tube 41 is fitted to the plug 52, and the tip 421 of the branch tube 42 is fitted to the plug 53.

The plugs 51, 52, 53 are each cylindrical in form, having an outer diameter that gradually decreases upward. In the illustrated embodiment, all of the plugs 51, 52, 53 protrude or extend in the same direction (i.e., upward as seen with reference to Figs. 4A and 4B.

By virtue of the configuration and positioning of the plugs 51, 52, 53, pulling the connector 5 toward the tip (leftward) in Fig. 2 in the state in which the plug 51 is connected to the first tube 3, the plug 52 is connected to the branch tube 41 and the plug 53 is connected to the branch tube 42 (hereinafter, this state is referred to as the fit state), the connector 5 can be removed from the tubes relatively rapidly and easily. Accordingly, the transition to the subsequent procedure (post-removal) involving connecting the first tube 3 to the catheter 10a, the branch tube 41 to the catheter 10b and the branch tube 42 to the catheter 10c is facilitated.

As shown in Fig. 2, the first cylinder 50, in the neighborhood of the plug 51, is provided with a first label (marking) 7 similar to the first label 7 on the first tube 3. This label 7 on the first cylinder can specify the tube that is to be connected to the plug 51 as the first tube 3. Accordingly, when forming an extracorporeal circuit by connecting the blood line 2 with the patient, upon removing the connector 5, the first tube 3 separated from the connector 5 can be connected relatively rapidly and positively to the catheter 10a previously positioned in the patient.

The first cylinder 50 is also provided, in the neighborhood of the plugs 52, 53, with respective second labels (markings) 8 similar to the second labels 8 on the branch tubes 41, 42. These labels can specify the tubes connected respectively to the plugs 52, 53, as the second tube(s) 4. Accordingly, when forming an extracorporeal circuit by connecting the blood line 2 with the patient, upon removing the connector 5, the branch tubes 41, 42 separated from the connector 5 can be connected, relatively rapidly and positively, to the respective catheters 10b, 10c previously positioned in the patient.

The plugs 51, 52, 53 are each provided with a respective through passage 510, 520, 530. The passages 510, 520, 530 open to and communicate with the lumen 500 of the first cylinder 50.

The second cylinder 54 is slidably received in the first cylinder 50 for sliding movement in the lengthwise direction of the first cylinder 50.

The second cylinder 54 is constructed as bottomed cylindrical member that is closed at one end (the base end) and open at the opposite end. The second cylinder 54 has a cylindrically shaped shell or wall similar to the first cylinder 50. The outer diameter of the second cylinder 54 is equal to or somewhat smaller than the inner diameter of the first cylinder 50.

The shell or wall of the second cylinder 54 is provided with two through-holes 541, 542 that are spaced apart from one another along the lengthwise direction of the second cylinder 54. The center-to-center distance between the through-holes 541, 542 is equal to the center-to-center distance between the passages 520, 530 in the connections or plugs 52, 53. The length of the second cylinder 54 is less than the length of the lumen 500 of the first cylinder.

This construction of the second cylinder 54 provides opening-and-closing means to open and close the passages 510, 520, 530 all together.

More specifically, as shown in Fig. 4A, when the tip of the second cylinder 54 is positioned on the base end side (i.e., right side) of the passage 510 (i.e., a state in which the passage 510 is not covered by the second cylinder 54) while the through-holes 541, 542 are respectively aligned with the passages 520, 530, the connector 5 is in a communication state in which the passages 510, 520, 530 communicate with the lumen 500. In the communication state, the passages 510, 520, 530 are communicated with one another through the internal space 500. This makes it possible to fill the first and second tubes 3, 4 with a priming liquid (physiological saline solution) from a priming liquid source.

When the second cylinder 54 is moved toward the tip relative to the first cylinder 50 (i.e., toward the left in Fig. 4A) and the tip of the second cylinder 54 abuts against the inner surface of the closed end of the first cylinder 50 as shown in Fig. 4B, the passages 510, 520, 530 are closed by the shell (wall) of the second cylinder 54. As a result, the connector 5 is in a non-communication state in which the passages 510, 520, 530 are not able to communicate with the lumen 500. In the non-communication state, the passages 510, 520, 530 are blocked from communicating one with another through the lumen 500. Accordingly, upon removing the connector 5 in the non-communication state from the first and second tubes 3, 4 that are filled with priming liquid following priming of the blood line 2, the priming liquid in the lumen 500 can be prevented from leaking to the outside.

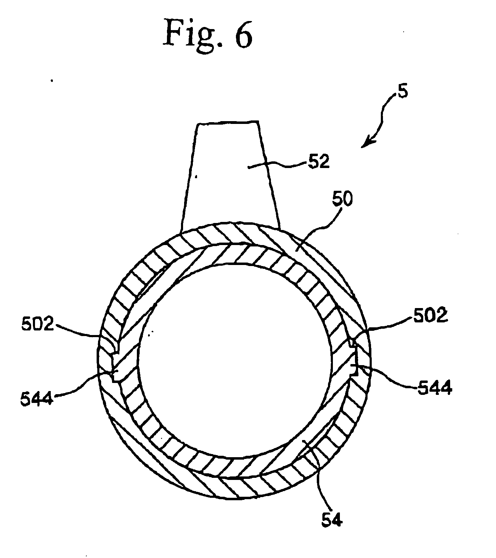

As shown in Figs. 5 and 6, two oppositely positioned (diametrically oppositely positioned) grooves or concave portions 502, 502 are provided in the inner surface of the first cylinder 50, intermediately with respect to the lengthwise extent of the first cylinder 50. The grooves 502, 502 extend in the lengthwise direction of the first cylinder 50. Two convex pieces or protrusions 544, 544 project outwardly from the outer surface of the second cylinder 54 at an intermediate position with respect to the lengthwise direction of the second cylinder. The convex pieces 544, 544 are positioned at opposite locations (diametrically opposite locations) on the second cylinder 54.

The convex pieces 544, 544 are respectively received in the grooves 502, 502 when the second cylinder 54 is positioned in the first cylinder 50 (i.e., the assembled state of the connector 5). By virtue of the positioning of the convex pieces 544, 544 in the grooves 502, 502, the convex pieces 544, 544 are guided in the respective grooves 502, 502 when the second cylinder 54 is slidably moved relative to the first cylinder 50. This can help prevent the second cylinder 54 from rotating relatively to the first cylinder 50.

In this manner, the grooves 502, 502 and the convex pieces 544, 544 serve as rotation preventing means that prevents the second cylinder 54 from rotating relative to the first cylinder 50. It is to be recognized that other forms of the rotation preventing means are possible. For example, the first and second cylinders 50, 54 can be made polygonal in cross-section to form rotation preventing means.

In the communication state, the second cylinder 54 is in a position closest to the tip end side of the first cylinder as shown in Fig. 5A. In this position, the convex pieces 544, 544 respectively abut against, or are in engagement with, the base ends of the grooves 502, 502 and so the second cylinder 54 is prevented from being unintentionally detached from the first cylinder 50. The grooves 502, 502 and the convex pieces 544, 544 thus serve also as falling-off preventing means that prevents the second cylinder 54 from falling off, or becoming separated from, the first cylinder 50.

As mentioned, the base end of the second cylinder 54 is provided with the manipulator 56. The manipulator 56 is mushroom-shaped comprising a grip (head) 561 for fingers to grasp and a connection 562 connecting the grip 561 to the second cylinder 54.

The connection 562 is configured as a cylindrical member having an outer diameter equal to the outer diameter of the second cylinder 54, it being understood that the term equal encompasses near equality. The outer surfaces of the second cylinder 54 and the connection 562 thus form together a continuous outer surface.

As shown in Figs. 4A, 4B, 5A and 5B, a label (visual confirmation means) 563 is provided on the connection 562. In the communication state of the connector 5, the label 563 is positioned outside the tip of the first cylinder 50 as shown in Figs. 4A and 5A. In the illustrated embodiment, the label 563 is positioned to immediately adjoin the tip of the first cylinder in the communication state of the connector 5 in which the convex portions 544, 544 abut the tip end side of the respective grooves 502, 502. In the non-communication state of the connector 5, the label 563 is positioned inside the first cylinder 50 as illustrated in Figs. 4B and 5B.

By visually confirming the position of the label 563, it is possible to positively know (visually confirm) the state of the connector 5, i.e., whether the connector 5 is in the communication state or the non-communication state.

The material forming the various parts of the connector 5 (i.e., the first cylinder 50, the plugs 51, 52, 53 and the second cylinder 54) is not limited. By way of example though, the material can be resin, e.g. polyvinyl chloride, polyethylene or polypropylene.

A method of connecting the blood line 2 with the catheters 10a, 10b, 10c positioned in the patient is as follows.

Initially, with the blood line assembly (the blood line 2 together with the connector 5) packaged in the package 6 as shown by way of example in Fig. 3, the blood line assembly is taken out of the package 6. That is, the package is opened and the first and second tubes 3, 4 are removed from the package together with the connector 5 which is connected to the tubes 3, 4. This can be accomplished by grasping the connector 5 shown in Fig. 3. In the Fig. 3 arrangement (i.e., when the connector 5 is in the packaging with the tubes 3, 4), the connector 5 is preferably in the communication state shown in Fig. 4A. After removing the blood line assembly from the package, the end of the tube 3 opposite the connector 5 is connected to the oxygenator 104, the end of the tube 4 opposite the connector 5 is connected to the reservoir 102, and the pump 101 is interposed between the reservoir 102 and the oxygenator. This thus forms a priming circuit. With the connector 5 in the communication state, priming is carried out. For example, priming liquid is introduced into the priming circuit so that the priming liquid from a priming liquid source flows through the tube 3, through the connector 5 in the communication state and through the tubing 4. Of course, during priming the priming liquid also flows through the blood reservoir 102, the tube 113, the oxygenator 104, the tube 114 and the pump 101. The priming liquid can be introduced into the priming circuit by connecting the source of priming liquid to the reservoir 102, for example in the manner shown in Fig. 12.

Once priming is completed, clamps (forceps) are respectively arranged around the tip 31 of the first tube 3, the tip 411 of the branch tube 41 and the tip 421 of the branch tube 42. This prevents the priming liquid from being discharged out of the tubes when the connector 5 is removed.

Thereafter, the connector 5 is removed from the first tube 3 and the branch tubes 41, 42. Prior to removing the tubes 3, 41, 42 from the connector 5, the second cylinder 54 is moved toward the base of the first cylinder 50 relative to the first cylinder 50, thus placing the connector 5 in the non-communication state shown in Fig. 4A. Priming liquid in the internal space 500 is thus not allowed to leak to the outside when the connector 5 is removed from the tubes 3, 4.

In an operation field, the catheters 10a, 10b, 10c are positioned in the patient. Upon removing the connector 5 from the tubes 3, 41, 42, the tip 31 of the first tube 3 and the tips 411, 412 of the branch tubes 41, 42 are able to be connected to the respective catheters 10a, 10b, 10c.

Then, the catheters 10a, 10b, 10c are respectively connected to the tip 31 of the first tube 3, the tip 411 of the branch tube 41 and the tip 421 of the branch tube 42. After this, the clamps applied to the tips of the tubes are removed.

The connector 5 described above and illustrated in the drawing figures is relatively simple in structure and can be relatively easily changed from the communication state to the non-communication state by a single-action manner. Advantageously, this does not impose a significant burden on the perfusionist. That is, the operation can be performed in a relatively simple way.

In this manner, in the blood line 2 disclosed here, the first and second tubes 3, 4 are able to be placed in a state connectable respectively with catheters 10a, 10b, 10c by merely removing the connector 5 from the first and second tubes 3, 4. In addition, the subsequent connections to the catheters 10a, 10b, 10c are relatively easy to perform, thus permitting a relatively rapid transition to extracorporeal circulation. Thus, the blood line 2 can be handled quite easily.

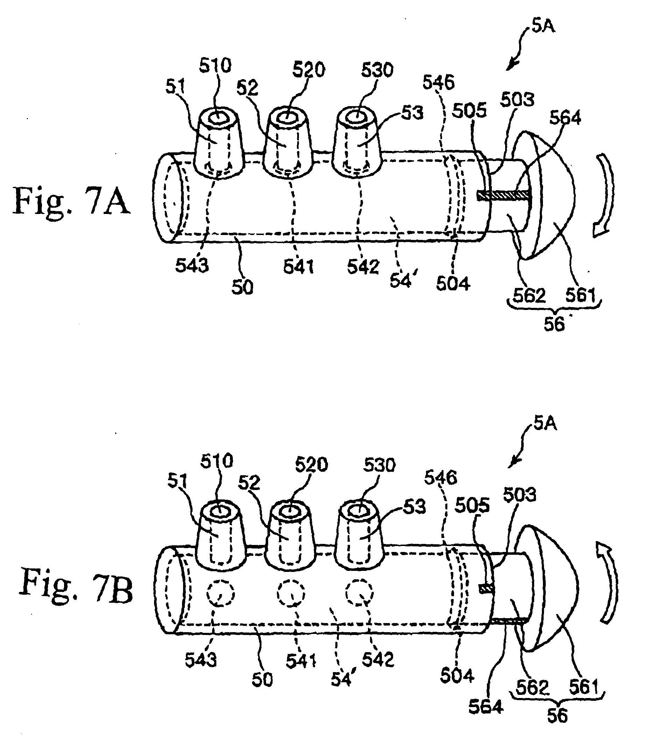

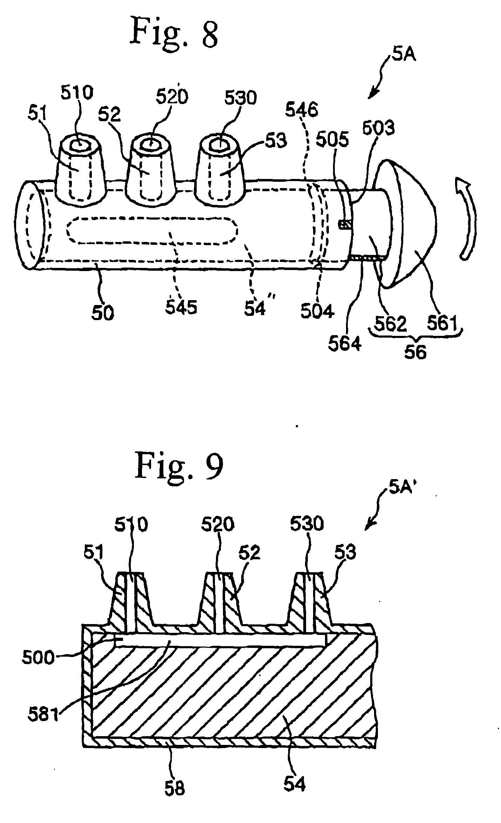

Figs. 7A and 7B are perspective views showing a second embodiment of a connector, with the communication state of the connector being shown in Fig. 7A and the non-communication state being shown in Fig. 7B. Fig. 8 illustrates an alternative configuration of the through-holes.

The description which follows primarily discusses features associated with the second embodiment of the connector and blood line that differ from the first embodiments described above. Features in the second embodiment of the connector that are the same as those in the first embodiment are identified by the same reference numerals and a detailed description of such features is not repeated. The second embodiment of the connector 5A differs from the first embodiment primarily in that the second cylinder 54' can be moved by rotation.

In the connector 5A shown in Figs. 7 A and 7B, the second cylinder 54' has a length equal to the length of the lumen 500 of the first cylinder 50, it being noted that the term equal encompasses near equality. The shell (wall) of the second cylinder 54' is provided with three through-holes 541, 542, 543 that are spaced apart from one another and, in the illustrated embodiment, arranged in a line.

In this embodiment, the communication state is obtained by aligning the through-holes 541, 542, 543 with respective ones of the passages 520, 530, 510 as shown in Fig. 7A. On the other hand, the non-communication state is obtained by rotating or displacing the second cylinder 54 about its longitudinal axis relative to the first cylinder 50 such that the through-holes 541, 542, 543 are not aligned with the passages 520, 530, 510 as shown in Fig. 7B.

As shown in Figs. 7A and 7B, a circumferentially extending groove or concave portion 504 is formed in the inner periphery (inner peripheral surface) of the first cylinder 50 at a position close to the opening 503 of the first cylinder 50. In the illustrated example, the concave portion 504 is triangular as seen in a vertical cross-section. However, the shape of the concave portion 504m is not limited in this regard and other shapes are possible such as a semicircle or the like.

The outer surface of the second cylinder 54' is provided with a circumferentially extending and outwardly projecting convex portion or projection 546. The convex portion 546 has a vertical cross-sectional form corresponding to the vertical cross-sectional form of the concave 504.

In the connector 5A, the convex portion 546 of the second cylinder 54' is fit or positioned in the concave portion or groove 504 of the first cylinder 50. By virtue of this arrangement, when rotating the second cylinder 54' relative to the first cylinder 50, the second cylinder 54' is prevented from unintentionally moving in the lengthwise direction relative to the first cylinder 50. In this way, the concave portion 504 and the convex portion 546 serve as movement preventing means that prevents the second cylinder 54 from axially moving relative to the first cylinder 50.

As shown in Figs. 7A and 7B, labels (visual confirmation means) 505, 564 are provided respectively on the outer peripheral surface of the first cylinder 50 and the outer peripheral surface of the connection 562 of the grip 56. In the communication state of the connector 5, the se labels 505, 564 are aligned with one another, thus provide a visual indication to the user of the communication state of the connector 5. That is, by visually confirming the positional relationship between the labels 505, 564, it is possible to positively view the condition of the connector 5A, namely whether the connector is in the communication state or the non-communication state.

In the illustrated example, the first and second cylinders 50, 54' have respective shells or walls that are cylindrical in form. However, the first and second cylinders can alternatively be a polygonal cylindrical form, e.g., octagon, in cross-sectional form. This can provide a click feeling while rotating the second cylinder 54 relative to the first cylinder 50, thus providing an indication that rotation is effected positively.

Fig. 8 illustrates an alternative to the through-holes 541, 542, 543 in the second cylinder. As illustrated in Fig. 8, the through-holes can be replaced by an elongated hole 545 in the second cylinder 54", thus constituting an arrangement in which the through-holes are all connected together as one elongated hole. The elongated hole 545 possesses a length at least equal to the distance between the passages 510, 530.

The connector 5A according to this second embodiment provides benefits and advantages similar to those described above in connection with the connector 5 of the first embodiment. In addition, a rotation-angle regulating means can be provided to regulate the rotation angle of the second cylinder 54' relative to the first cylinder 50.

Fig. 9 is a longitudinal cross-sectional view showing a third embodiment of a connector disclosed herein. The description which follows primarily discusses features associated with the third embodiment of the connector and blood line that differ from the first and second embodiments described above. Features in the third embodiment of the connector that are the same as those in the embodiments described above are identified by the same reference numerals and a detailed description of such features is not repeated. The connector 5A' in this embodiment is similar to that of the second embodiment, except that the second cylinder is replaced by a reception member of solid form positioned in the first cylinder 50.

The connector 5A' shown in Fig. 9 includes a solid reception member or movable member 58, generally corresponding to the second cylinder or movable member 54 described in the second embodiment, that is received in the first cylinder 50. The outer periphery of the reception member 58 includes a communication passage or groove 581 located at a position corresponding to the passages 510, 520, 530. The communication passage 581 extends in the lengthwise direction of the reception member 58.

By positioning the communication passage 581 underneath the passages 510, 520, 530, the connector 5A' is placed in a communication state, as shown in Fig. 9. By rotating the receiving member 58, the communication passage 581 is separated from the passages 510, 520, 530, thus placing the connector 5A' in a non-communication state.

During priming of the blood line, the connector 5A' requires a smaller amount of priming liquid than that associated with using the connector 5A of the second embodiment.

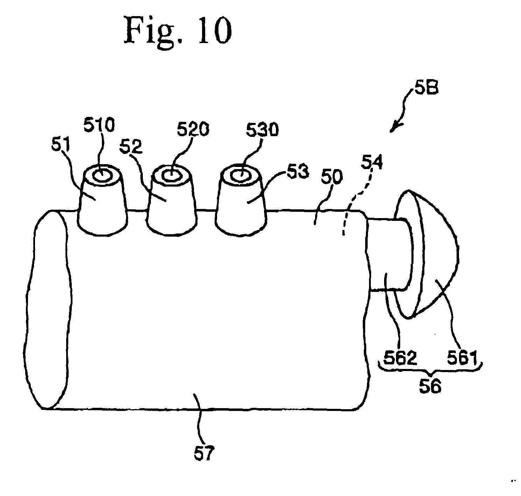

Fig. 10 is a perspective view of a fourth embodiment of a connector disclosed herein. The description which follows primarily discusses features associated with the fourth embodiment of the connector and blood line that differ from the first, second and third embodiments described above.
Features in the fourth embodiment of the connector that are the same as those in the embodiments described above are identified by the same reference numerals and a detailed description of such features is not repeated.

The connector 5B in this embodiment is similar to those of the first, second and third embodiments except that the connector is provided with a grip portion 57. The grip portion 57 constitutes a portion of the connector which is gripped by the user when attaching/detaching the connector 5B to/from the tubes. The grip portion 57 has a rounded exterior surface. This makes it relatively easy to hold the grip portion 57 and hence to attach/detach the connector 5. The grip 57 may also be in a ring form.

The connector 5B of the fourth embodiment can achieve benefits and advantages similar to the connectors 5, 5A of the earlier embodiments.

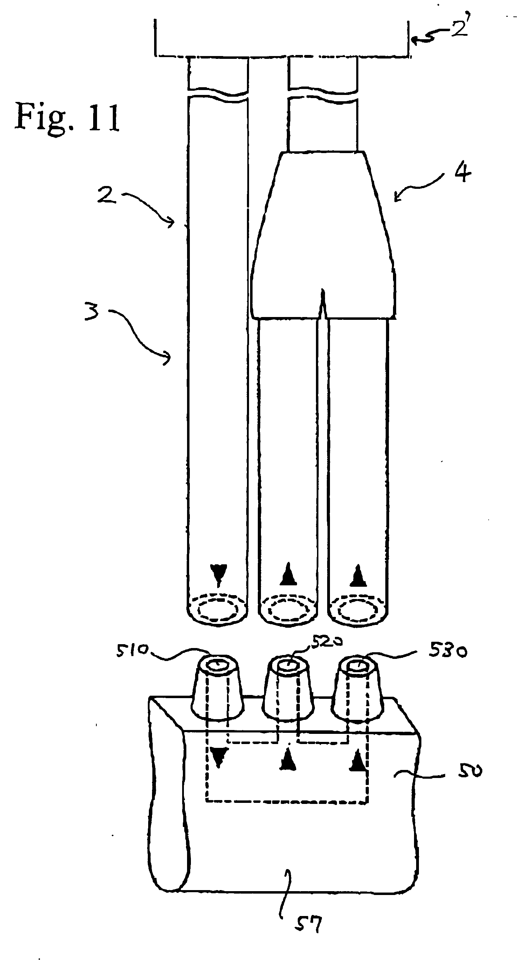

Fig. 11 shows a fifth embodiment of the connector and blood line.
In this embodiment, the second cylinder 54 and the manipulator 56 of the fourth embodiment are eliminated, and the passages 510, 520, 530 are in mutual communication with one another within a cylinder 50.

The combination of the connecter and blood line, as described above and illustrated in the various drawing figures, together form a blood line assembly.

The connector and blood line disclosed here is illustrated and described in connection with the various embodiments. However, the invention is not limited to the disclosed elements, and the structure of the blood line and connector can be varied, and other features can be added.

For example, the connector's connections are not limited to projections or plugs (structured as a convex element), or a male configuration, but may be, for example a concave element providing a female configuration. Also, the connections of the connector are not limited to three in number as two or four (or more) may be provided.

The connector may be provided with state-maintaining means that maintains the connector in the communication or non-communication state.

Also, the connector and the blood line may be a combination of two or more structures (features) from amongst the foregoing embodiments.

The first and second labels may be the same color or different in color. In the described embodiments, the first and second labels are provided on both the first and second tubes and the connectors. However, the connector and blood line are not limited in this regard as they may be placed on only one of those.

Further, the blood reservoir may be replaced with a bubble removing device that removes bubbles from a circuit, and the centrifugal pump may be replaced with a roller pump.

Thus, it is to be understood that the principles, preferred embodiments and other disclosed aspects have been described in the foregoing specification. However, the invention which is intended to be protected is not to be construed as limited to the particular embodiments disclosed. Further, the embodiments described herein are to be regarded as illustrative rather than restrictive. Variations and changes may be made by others, and equivalents employed, without departing from the spirit of the present invention. Accordingly, it is expressly intended that all such variations, changes and equivalents which fall within the spirit and scope of the present invention as defined in the claims, be embraced thereby.

## Claims

1. A blood line assembly comprising:
a first tube possessing an end;
a second tube comprised of a second tube body possessing an end and two branch tubes branching from the end of the second tube body, each of the two branch tubes possessing an end;
a connector comprising first, second and third connections each provided with a through passage, the end of the first tube being removably fitted to the first connection, the end of each of the branch tubes being removably fitted to the second and third connections respectively; and
the first tube and the branch tubes being removable from the respective connections of the connector for subsequent connection to respective catheters positioned in a patient.

2. A blood line assembly according to Claim 1, wherein the connector comprises an internal space, and wherein the connections communicate with one another through the internal space.

3. A blood line assembly according to Claim 1, wherein the connector comprises a cylinder possessing a closed end, an open end and an interior space, the connections extending outwardly from the cylinder, and wherein the connector also comprises a movable member movably positioned within the cylinder.

4. A blood line assembly according to Claim 3, wherein the cylinder is a first cylinder and the movable member is a second cylinder comprising at least two through holes spaced apart a distance equal to a spacing between the passages of two of the connections.

5. A blood line assembly according to Claim 3, wherein the second connection is positioned between the first and third connections, and wherein the cylinder is a first cylinder and the movable member is a second cylinder comprising an elongated through hole possessing a length at least equal to the distance between the passages of the first and third connections.

6. A blood line assembly according to Claim 3, wherein the second connection is positioned between the first and third connections, and wherein the movable member is a solid reception member possessing an outer surface provided with a groove, the groove having a length at least equal to a distance between the passages of the first and third connections.

7. A blood line assembly according to Claim 3, wherein the movable member and the cylinder are provided with respective markings adapted to be aligned with one another to indicate a relative rotation position between the movable member and the cylinder.

8. A blood line assembly according to Claim 3, wherein the movable member is provided with at least one through hole, and wherein the movable member and the cylinder are provided with respective markings adapted to be aligned with one another to indicate a relative rotation position between the movable member and the cylinder in which the passage of one of the connections is rotationally aligned with the at least one through hole in the movable member.

9. A blood line assembly according to Claim 3, further comprising a groove that receives a projection, the groove being provided on either the cylinder or the movable member and the projection being provided on the other of the cylinder and the movable member.

10. A blood line assembly according to Claim 1, wherein the first tube, the second tube and the connector are packaged in a package in a condition in which the end of the first tube is removably fitted to the first connector, and the end of each of the branch tubes is removably fitted to the second and third connectors respectively.

11. A connector removably connectable to a blood line communicating with several medical devices to facilitate a priming operation comprising:
a connector body possessing a lumen;
a plurality of connections provided on the connector body, each connector possessing a through passage communicating with the lumen of the connector body, each of the connections being adapted to be removably connected to a respective tube to communicate a lumen of each tube to the lumen of the connector body;
opening-and-closing means for opening and closing the passages of all of said plurality of connections together; and
the opening-and-closing means being operable to selectively position the connector in a communication state in which the passages of all of said plurality of connections are in communication with the lumen and a non-communication state in which the passages of all of said plurality of connections are blocked from communicating with the lumen.

12. A connector according to Claim 11, wherein:
the connector body is a first cylinder closed at one end and open at an opposite end; and
the opening-and-closing means comprises a second cylinder closed at one end, positioned in the lumen and movable relative to the first cylinder between the non-communication state in which a wall of the second cylinder closes the passages and the communication state.

13. A connector according to Claim 12, wherein the second cylinder is movable relative to the first cylinder in a lengthwise direction of the first cylinder.

14. A connector according to Claim 13, wherein:
the plurality of connections are spaced apart along a lengthwise direction of the first cylinder;
the plurality of connections comprises first, second and third connections each provided with a respective passage, with the second connection being located between the first and third connections; and
a plurality of through-holes pass through the wall of the second cylinder, the plurality of through-holes being one less in total number than a total number of connections.

15. A connector according to Claim 12, wherein the second cylinder is rotatably movable about a longitudinal axis of the first cylinder relative to the first cylinder.

16. A connector according to Claim 15, wherein a plurality of through-holes pass through the wall of the second cylinder at spaced apart positions corresponding to positions of the passages in the connections when the second passages and the through-holes are rotationally aligned.

17. A method of using a blood line assembly comprising:
communicating a blood line assembly with a source of priming liquid, the blood line assembly comprising a connector, a first tube and a second tube, one of the first and second tubes being comprised of a tube body and two branch tubes branching from the tube body, the connector comprising first, second and third connections each provided with a through passage, with the first tube being removably fitted to the first connection and each of the branch tubes being removably fitted to the second and third connections respectively;
introducing the priming liquid from the priming liquid source into the first tube so that the priming liquid in the first tube flows into the second tube by way of the connector;
disconnecting the first tube from the first connection and disconnecting each of the branch tubes from the second and third connections respectively; and
connecting the first tube and the branch tubes to a respective catheter positioned in a patient.

18. A method according to Claim 17, wherein the priming liquid in the first tube flows into the second tube by way of the connector when the connector is in a communication state, and further comprising changing the connector to a non-communication state in which the passages of the first, second and third connections are blocked to prevent communication between the first tube and the second tube, the connector being changed to the non-communication state before disconnecting the first tube from the first connection and before disconnecting each of the branch tubes from the second and third connections respectively.

19. A method according to Claim 17, wherein the connector comprises a cylinder from which the first, second and third connections outwardly extend, the connector further comprising a movable member positioned within the cylinder, the connector being changed from the communication state to the non-communication state by rotating the movable member.

20. A method according to Claim 17, wherein the connector comprises a cylinder from which the first, second and third connections outwardly extend, the connector further comprising a movable member positioned within the cylinder, the connector being changed from the communication state to the non-communication state by slidably moving the movable member within the cylinder in a direction along a longitudinal extent of the cylinder.

21. A method according to Claim 17, wherein the priming liquid source is connected to a reservoir in communication with the first tube, and the priming liquid from the priming liquid source is introduced into the first tube by way of the reservoir.

22. A method according to Claim 17, wherein the second tube communicates with an oxygenator, and wherein the priming liquid in the second source flows into the oxygenator.
